# EUROPEAN PATENT APPLICATION

(11) **EP 1 460 058 A1**
(43) Date of publication of application: **22.09.2004**
(21) Application number: 02788804.9
(22) Date of filing: 12.12.2002
(51) Int. Cl.: C07C 229/48, C07C 227/04, C07C 233/58

(54) **DIAMINODICARBOXYLIC ACIDS AND INTERMEDIATES THEREOF**

(30) Priority: 27.12.2001 JP 2001397246
(71) Applicant: Mitsui Chemicals, Inc., Tokyo 105-7117 (JP)
(72) Inventor: MITA, Naruyoshi, Sodegaura-shi, Chiba 299-0265 (JP); CHIDA, Mitsuaki, Sodegaura-shi, Chiba 299-0265 (JP); ISOKAWA, Motoaki, Sodegaura-shi, Chiba 299-0265 (JP); KATOU, Takazou, Sodegaura-shi, Chiba 299-0265 (JP); NAGAI, Naoshi, Sodegaura-shi, Chiba 299-0265 (JP)
(74) Representative: Stuart, Ian Alexander
(86) International application number: PCT/JP2002/012993
(87) International publication number: WO 2003/055846

(57) **Abstract**

The present invention relates to a novel diaminodicarboxylic acid represented by general formula (1): 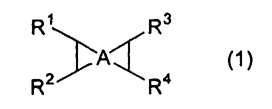 (wherein A is an aliphatic group; one of R¹ and R² is a carboxyl group, and the other is an amino group or a carbamoyl group; and one of R³ and R⁴ is a carboxyl group, and the other is an amino group or a carbamoyl group), a carbamoyl compound which is a synthetic intermediate for the diaminodicarboxylic acid, and a process for producing the diaminodicarboxylic acid.

The diaminodicarboxylic acid can be produced by Hofmann reaction of the carbamoyl compound and is a useful compound as a starting material for resins or an additive for resins.

## Description

### Technical Field

The present invention relates to novel diaminodicarboxylic acids and their salts which are useful as starting materials for resins and additives for resins. The present invention also relates to synthetic intermediates for diaminodicarboxylic acids and processes for producing diaminodicarboxylic acids.

### Background Art

Toward the realization of an advanced information society, technologies have been remarkably developed in the semiconductor, optical communication, display, aerospace, and other fields. There have been demands for increases in performance and functionality of materials used. In order to meet such demands, starting materials for resins and additives having physical properties, such as heat resistance, optical characteristics, mechanical characteristics, electrical characteristics, chemical stability, low hygroscopicity, and high formability, are now being developed. However, satisfactory compounds have not been found yet.

For example, polyimide resins prepared by reaction of tetracarboxylic dianhydrides with diamines are widely used as base materials because of their excellent heat resistance, flame retardance, mechanical strength, electrical insulation, etc. However, the existing polyimides do not satisfactorily provide optical and other characteristics which are newly required in the resins used for electronic materials and resins used for electronic information materials. Accordingly, development of novel starting materials for polyimides which can satisfy such requirements has been desired.

On the other hand, although examples of syntheses of aromatic diaminodicarboxylic acids have been reported in Bull. Chem. Soc. Jpn., 40, 2429(1967), etc., examples of syntheses of aliphatic diaminodicarboxylic acids and cycloaliphatic diaminodicarboxylic acids which are useful as starting materials for resins and additives have not been reported yet.

### Disclosure of Invention

It is an object of the present invention to provide a novel diaminodicarboxylic acid which can be used as a starting material for resins or an additive for resins to improve heat resistance, optical characteristics, mechanical characteristics, electrical characteristics, chemical stability, hygroscopicity, formability, etc., and in particular, which is useful as a starting material for polyimides. It is another object of the present invention to provide a synthetic intermediate for the diaminodicarboxylic acid and a process for producing the diaminodicarboxylic acid.

The present inventors have carried out thorough research to achieve the objects described above and have found a novel aliphatic group-containing diaminodicarboxylic acid, a novel dicarbamoyldicarboxylic acid which is a synthetic intermediate for the diaminodicarboxylic acid, and a process for producing the diaminodicarboxylic acid, thus completing the present invention.

That is, the present invention includes the following Items.
[1] A compound represented by general formula (1) or a salt thereof: 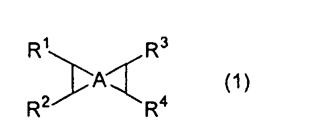 wherein A is a tetravalent aliphatic group having 2 to 20 carbon atoms which may contain at least one of a halogen atom, oxygen atom, and sulfur atom; one of R¹ and R² is a carboxyl group, and the other is an amino group or a carbamoyl group (-CONH₂); and one of R³ and R⁴ is a carboxyl group, and the other is an amino group or a carbamoyl group.
[2] A compound or a salt thereof according to Item [1], wherein, in general formula (1), one of R¹ and R² is a carboxyl group, and the other is an amino group; and one of R³ and R⁴ is a carboxyl group, and the other is an amino group.
[3] A compound or a salt thereof according to Item [1], wherein, in general formula (1), one of R¹ and R² is a carboxyl group, and the other is a carbamoyl group; and one of R³ and R⁴ is a carboxyl group, and the other is a carbamoyl group.
[4] A compound or a salt thereof according to any one of Items [1] to [3], wherein, in general formula (1), the group represented by: 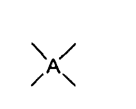 is any one of a group represented by general formula (2): 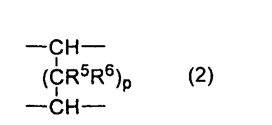 wherein each of R⁵ and R⁶ is independently a hydrogen atom, a halogen atom, or an alkyl group having 1 to 6 carbon atoms; R⁵ and R⁶ may be linked together to form a divalent hydrocarbon group; each of the alkyl group and the divalent hydrocarbon group may be substituted with a halogen atom; and p is an integer of 1 to 3,
   a group represented by general formula (3): 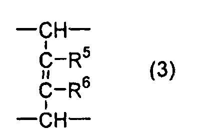 wherein R⁵ and R⁶ are as defined in general formula (2), and a group represented by general formula (4): 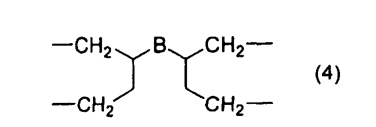 wherein B is a single bond, a divalent hydrocarbon group having 1 to 12 carbon atoms, an oxygen atom, a sulfur atom, -CO-, or -SO₂-; and the divalent hydrocarbon group may be substituted with a halogen atom.
[5] A process for producing a diaminodicarboxylic acid represented by general formula (6) or a salt thereof: 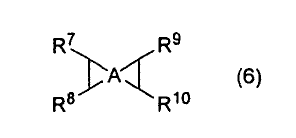 wherein A is a tetravalent aliphatic group having 2 to 20 carbon atoms which may contain at least one of a halogen atom, oxygen atom, and sulfur atom; one of R⁷ and R⁸ is a carboxyl group, and the other is an amino group; and one of R⁹ and R¹⁰ is a carboxyl group, and the other is an amino group, the process comprising the steps of:
   reacting an acid dianhydride represented by general formula (5): 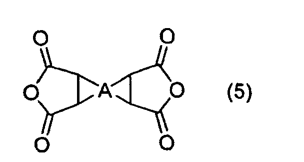
wherein A is as defined in general formula (6), with ammonia; and
subjecting the reaction product to Hofmann rearrangement.

### Best Mode for Carrying Out the Invention

The present invention will be described in detail below.

In the compound represented by general formula (1), one of R¹ and R² is a carboxyl group, and the other is an amino group or a carbamoyl group; and one of R³ and R⁴ is a carboxyl group, and the other is an amino group or a carbamoyl group.

In general formula (1), examples of the tetravalent aliphatic group represented by A having 2 to 20 carbon atoms include saturated aliphatic groups and unsaturated aliphatic groups, and also include straight-chain, branched, and cyclic aliphatic groups. These groups may contain at least one of a halogen atom, oxygen atom, and sulfur atom.

Preferred examples of the group represented by A include the groups represented by general formulae (2), (3), and (4). More specifically, compounds having these groups are respectively represented by the following general formulae (7), (8), and (9):

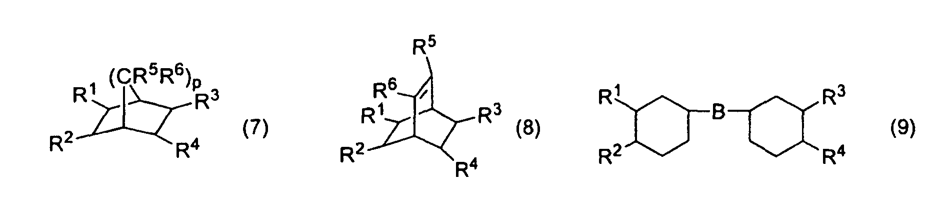

In general formulae (2) and (3), examples of the halogen atom for each of R⁵ and R⁶ include a fluorine atom, a chlorine atom, a bromine atom, and an iodine atom.

The alkyl group having 1 to 6 carbon atoms for each of R⁵ and R⁶ may be a straight-chain, branched, or cyclic alkyl group, and examples thereof include a methyl group, an ethyl group, an n-propyl group, an isopropyl group, an n-butyl group, an isobutyl group, a sec-butyl group, a tert-butyl group, a pentyl group, a hexyl group, a cyclopentyl group, and a cyclohexyl group.

Examples of the alkyl group substituted with a halogen atom for each of R⁵ and R⁶ include a trifluoromethyl group.

The divalent hydrocarbon group formed by the linkage of R⁵ and R⁶ may be a straight-chain or branched hydrocarbon group, and examples thereof include an ethylene group, a trimethylene group, a tetramethylene group, and a pentamethylene group. When such a divalent hydrocarbon group is substituted with a halogen atom, the position of the substitution by the halogen atom is not particularly limited.

In general formula (2), p is an integer of 1 to 3 and preferably 1 to 2.

In general formula (4), the divalent hydrocarbon group having 1 to 12 carbon atoms for B may be a straight-chain, branched, or cyclic hydrocarbon group, and examples thereof include a methylene group, an ethylene group, a trimethylene group, a tetramethylene group, a dimethylmethylene group, and a cyclohexylene group.

Examples of the divalent hydrocarbon group substituted with a halogen atom for B include a difluoromethylene group and a bis(trifluoromethyl)methylene group.

The compound represented by general formula (1) has stereoisomers based on the steric relationships between R¹, R², R³, and R⁴. The compound may be composed of one stereoisomer or a mixture of a plurality of stereoisomers.

When the compound represented by general formula (1) is a diaminodicarboxylic acid, examples of the salt of the compound include an ammonium salt formed by reaction of the amino group of the diaminodicarboxylic acid with an acid and a carboxylate salt formed by reaction of the carboxyl group of the diaminodicarboxylic acid with a base. When the compound represented by general formula (1) is a dicarbamoyldicarboxylic acid, examples of the salt of the compound include a carboxylate salt formed by reaction of the carboxyl group of the dicarbamoyldicarboxylic acid with a base.

The ammonium salt may be a salt of an inorganic acid or a salt of an organic acid.

Examples of the inorganic acid include hydrochloric acid, hydrogen bromide, hydrogen iodide, sulfuric acid, carbonic acid, and phosphoric acid.

The organic acid may be optically active or optically inactive, and examples thereof include carboxylic acids, such as formic acid, acetic acid, propionic acid, benzoic acid, trifluoroacetic acid, tartaric acid, and mandelic acid; sulfonic acids, such as methanesulfonic acid, ethanesulfonic acid, benzenesulfonic acid, and p-toluenesulfonic acid; and amino acids and their derivatives.

Examples of the carboxylate salt formed by reaction of the carboxyl group of the compound represented by general formula (1) with a base include metal salts and nonmetal salts of carboxylic acids.

Examples of the metal salts include lithium salts, sodium salts, potassium salts, magnesium salts, calcium salts, aluminum salts, iron salts, silver salts, copper salts, and mercury salts.

Examples of the nonmetal salts include ammonium salts, dimethylammonium salts, trimethylammonium salts, triethylammonium salts, and pyridinium salts.

In order to form the salt, the compound of the present invention and the acid or base may be used in equivalent amounts or in any composition ratio.

### [Process for producing dicarbamoyldicarboxylic acid and salt thereof]

A process for producing a dicarbamoyldicarboxylic acid (i.e., a compound represented by general formula (1), wherein one of R¹ and R² is a carboxyl group, and the other is a carbamoyl group; and one of R³ and R⁴ is a carboxyl group, and the other is a carbamoyl group), and a salt thereof will be described below.

The dicarbamoyldicarboxylic acid of the present invention can be produced by reacting the acid dianhydride represented by general formula (5) with ammonia.

Examples of ammonia include aqueous ammonia, ammonia gas, and liquid ammonia.

When aqueous ammonia is used, besides commercially available aqueous ammonia with an ammonia concentration of 10% by weight, 25% to 28% by weight, or 28% to 30% by weight, aqueous ammonia with an ammonia concentration appropriately adjusted in the range from 1% by weight to the saturated concentration at the reaction temperature may be used.

When ammonia gas is used, preferably, ammonia gas is introduced into a reaction solvent. Alternatively, the reaction may be carried out in an ammonia atmosphere (under ammonia flow). Besides commercially available ammonia gas (99.9% to 100%), ammonia in a mixture with nitrogen gas, argon gas, air, or the like may be used.

When aqueous ammonia or liquid ammonia is employed, an organic solvent may be used, or each may be used as a solvent without using an organic solvent.

Ammonia is used in an amount of not less than 2 molar times and preferably not less than 5 molar times the amount of the acid dianhydride represented by general formula (5).

Any solvent which does not affect the reaction of the acid dianhydride with ammonia may be used. Examples of solvents include water; saturated hydrocarbons, such as pentane, hexane, heptane, and cyclohexane; aromatic hydrocarbons, such as benzene, toluene, xylene, and ethyl benzene; halogenated hydrocarbons, such as dichloromethane, chloroform, carbon tetrachloride, dichloroethane, chlorobenzene, and dichlorobenzene; lower alcohols, such as methanol, ethanol, 1-propanol, isopropyl alcohol, and tert-butyl alcohol; glycols, such as ethylene glycol, diethylene glycol, and propylene glycol; ethers, such as ethylene glycol dimethyl ether, 1,3-dioxane, 1,4-dioxane, tetrahydrofuran, dimethyl ether, diethyl ether, diisopropyl ether, and dibutyl ether; amides, such as formamide and N,N-dimethylformamide; nitriles, such as acetonitrile; ketones, such as acetone and methyl ethyl ketone; esters, such as methyl acetate and ethyl acetate; sulfur-containing solvents, such as dimethyl sulfoxide; and 1,3-dimethyl-2-imidazolidinone.

These solvents may be used alone or in combination of two or more. When used as a mixture, any mixing ratio is acceptable. Additionally, the acid dianhydride, i.e., the starting material, may be subjected to reaction after being dissolved in a reaction solvent or in a slurry state.

The amount of the reaction solvent used is not particularly limited, but is 1 to 1,000 g, preferably 1 to 500 g, and more preferably 1 to 100 g relative to 1 g of the acid dianhydride, the starting material.

When a mixture of water and an organic solvent that is immiscible with water is selected as a reaction solvent, a phase transfer catalyst, such as a tetrabutyl ammonium salt (e.g., tetrabutylammonium hydrogensulfate), may also be used.

When liquid ammonia is used as a solvent, the reaction temperature ranges from -77°C to -33°C. When a mixture of liquid ammonia and other solvent is used, the reaction temperature ranges from -77°C to the boiling point of the solvent.

When aqueous ammonia or ammonia gas is used, the reaction is carried out in a temperature range from -20°C to the boiling point of the solvent. When the reaction is carried out with ammonia gas being injected into a reaction solvent, ammonia may be absorbed by the reaction solvent at -20°C to 50°C in advance before the temperature is raised to the reaction temperature, or ammonia may be absorbed by the reaction solvent at the reaction temperature. The reaction may be carried out by the combination of these operations.

Additionally, when the reaction is carried out in an autoclave, regardless of the form of ammonia, the reaction temperature is not limited to the ranges described above, and the reaction temperature ranges from -20°C to 200°C and preferably from -20°C to 150°C.

The reaction time is not particularly limited and is set appropriately depending on the starting material, the reaction conditions, etc. The reaction time is usually 10 minutes to 48 hours.

In this reaction, an imide form represented by general formula (10) or a salt thereof: 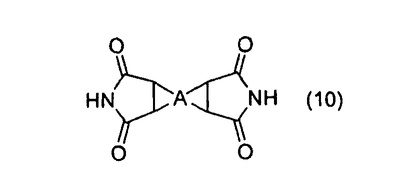 wherein A is as defined previously, may be produced. In such a case, the imide can be transformed into the dicarbamoyldicarboxylic acid of the present invention by the conventional method for hydrolysis.

The isolation method for the dicarbamoyldicarboxylic acid of the present invention is not particularly limited. When the product is precipitated from the reaction solvent, isolation can be performed by filtration or centrifugal separation. When the product is dissolved in the reaction solvent, removal of the solvent by distillation under reduced pressure or precipitation of the product by addition of a proper solvent followed by filtration or centrifugal separation may be employed as the isolation method. Alternatively, a carboxylate salt may be formed by treatment with a proper base before carrying out the operation described above. The isolation may be carried out by the combination of these methods.

When it is necessary to purify the dicarbamoyldicarboxylic acid of the present invention, any well-known conventional method may be employed, for example, distillation, recrystallization, column chromatography, sludge treatment, and activated carbon treatment.

The solvent used for recrystallization, column chromatography, sludge treatment, and activated carbon treatment is not particularly limited. Examples of solvents include water; saturated hydrocarbons, such as pentane, hexane, heptane, and cyclohexane; aromatic hydrocarbons, such as benzene, toluene, xylene, and ethyl benzene; halogenated hydrocarbons, such as dichloromethane, chloroform, carbon tetrachloride, dichloroethane, chlorobenzene, and dichlorobenzene; lower alcohols, such as methanol, ethanol, 1-propanol, isopropyl alcohol, and tert-butyl alcohol; glycols, such as ethylene glycol, diethylene glycol, and propylene glycol; ethers, such as ethylene glycol dimethyl ether, 1,3-dioxane, 1,4-dioxane, tetrahydrofuran, dimethyl ether, diethyl ether, diisopropyl ether, and dibutyl ether; amides, such as formamide and N,N-dimethylformamide; nitriles, such as acetonitrile; ketones, such as acetone and methyl ethyl ketone; carboxylic acids, such as formic acid and acetic acid; esters, such as methyl acetate and ethyl acetate; sulfur-containing solvents, such as dimethyl sulfoxide; and 1,3-dimethyl-2-imidazolidinone.

Additionally, aqueous ammonia, hydrochloric acid, an aqueous solution of acetic acid, or the like may be used as water.

These solvents may be used alone or in combination of two or more. When used as a mixture, any mixing ratio is acceptable.

In recrystallization, sludge treatment, or activated carbon treatment, the amount of the solvent used is 1 to 1,000 g and preferably 1 to 300 g relative to 1 g of the dicarbamoyldicarboxylic acid.

### [Process for producing diaminodicarboxylic acid and salt thereof]

A process for producing a diaminodicarboxylic acid (i.e., a compound represented by general formula (1), wherein one of R¹ and R² is a carboxyl group, and the other is an amino group; and one of R³ and R⁴ is a carboxyl group, and the other is an amino group), and a salt thereof will now be described below.

The diaminodicarboxylic acid represented by general formula (6) corresponds to the diaminodicarboxylic acid described above.

The diaminodicarboxylic acid of the present invention can be produced by reacting the acid dianhydride represented by general formula (5) with ammonia and then carrying out Hofmann rearrangement.

Herein, Hofmann rearrangement is a reaction in which an amide form or imide form gives a primary amine containing one carbon atom less than the original amide form or imide form, the reaction involving the migration of an alkyl group or aryl group from carbon to nitrogen.

The reaction of the acid dianhydride with ammonia in the first stage is carried out as in the process for producing the dicarbamoyldicarboxylic acid and the salt thereof described above, and thereby the dicarbamoyldicarboxylic acid (amide form) represented by general formula (1) or the imide form represented by general formula (10) is produced. Additionally, when the compound is isolated as a salt (e.g., sodium salt or potassium salt) thereof, the salt may be used as it is in the subsequent reaction or the salt may be subjected to desalting in accordance with the conventional method before use in the subsequent reaction.

In Hofmann rearrangement in the second stage, any reagent which causes Hofmann rearrangement may be used. Known examples thereof include hypochlorites, hypobromites, and organic iodine compounds, such as I,I-bis(trifluoroacetoxy)iodobenzene and I,I-bis(acetoxy)iodobenzene.

Examples of hypochlorites and hypobromites include sodium hypochlorite, potassium hypochlorite, sodium hypobromite, and potassium hypobromite. These may be prepared in the reaction solution using chlorine and sodium hydroxide, chlorine and sodium methoxide, chlorine and potassium hydroxide, bromine and sodium hydroxide, bromine and sodium methoxide, bromine and potassium hydroxide, and the like.

The reagent is used in an amount of 1 to 20 molar times, preferably 1.4 to 10 molar times, and more preferably 1.5 to 5 molar times the amount of the amide form represented by general formula (1) or the imide form represented by general formula (10). Additionally, when an aqueous hypochlorite solution is used, the amount of the aqueous hypochlorite solution is not limited to the above ranges, and the aqueous hypochlorite solution may be used as the reaction solvent.

Any solvent which is inactive against the starting material and the product may be used in Hofmann rearrangement. Examples of solvents include water; saturated hydrocarbons, such as pentane, hexane, heptane, and cyclohexane; aromatic hydrocarbons, such as benzene, toluene, xylene, and ethyl benzene; halogenated hydrocarbons, such as dichloromethane, chloroform, carbon tetrachloride, dichloroethane, chlorobenzene, and dichlorobenzene; lower alcohols, such as methanol, ethanol, 1-propanol, isopropyl alcohol, and tert-butyl alcohol; glycols, such as ethylene glycol, diethylene glycol, and propylene glycol; ethers, such as ethylene glycol dimethyl ether, 1,3-dioxane, 1,4-dioxane, tetrahydrofuran, dimethyl ether, diethyl ether, diisopropyl ether, and dibutyl ether; amides, such as formamide and N,N-dimethylformamide; nitriles, such as acetonitrile; ketones, such as acetone and methyl ethyl ketone; esters, such as methyl acetate and ethyl acetate; sulfur-containing solvents, such as dimethyl sulfoxide; and 1,3-dimethyl-2-imidazolidinone.

Additionally, a basic aqueous solution, such as an aqueous sodium hydroxide solution or an aqueous potassium hydroxide solution, may be used as water.

These solvents may be used alone or in combination of two or more. When used as a mixture, any mixing ratio is acceptable. Additionally, the amide form or imide form, i.e., the starting material, may be subjected to reaction after being dissolved in a reaction solvent or in a slurry state.

The amount of the reaction solvent used is not particularly limited, but is 1 to 1,000 g and preferably 1 to 100 g relative to 1 g of the amide form or imide form, the starting material.

When a mixture of water and an organic solvent that is immiscible with water is selected as a reaction solvent, a phase transfer catalyst, such as a tetrabutyl ammonium salt (e.g., tetrabutylammonium hydrogensulfate), may also be used.

The reaction temperature usually ranges from -20°C to the boiling point of the solvent.

The reaction time is not particularly limited and is set appropriately depending on the starting material, the reaction conditions, etc. The reaction time is usually 10 minutes to 48 hours.

In this reaction, an isocyanic ester represented by general formula (11): 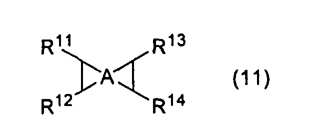 (wherein A is as defined previously; one of R¹¹ and R¹² is an isocyanato group (-NCO), and the other is a carboxyl group; and one of R¹³ and R¹⁴ is an isocyanato group, and the other is a carboxyl group), is formed as an intermediate. The isocyanic ester may be isolated. When the reaction solvent does not contain water, the isocyanic ester is the product.

The resultant isocyanic ester represented by general formula (11) can be transformed into the diaminodicarboxylic acid of the present invention by stirring the isocyanic ester in the presence of an acid, such as hydrochloric acid, sulfuric acid, phosphoric acid, acetic acid, trifluoroacetic acid, or p-toluenesulfonic acid, or a base, such as sodium hydroxide or potassium hydroxide, and water at room temperature to 100°C for 10 minutes to 120 hours.

In such a case, the organic solvent exemplified above with respect to Hofmann rearrangement may also be used. When a mixture of water and an organic solvent that is immiscible with water is selected, a phase transfer catalyst, such as a tetrabutyl ammonium salt (e.g., tetrabutylammonium hydrogensulfate), may also be used. The reaction may be carried out in an autoclave at 100°C to 200°C.

Furthermore, the isocyanic ester can be transformed into the diaminodicarboxylic acid by a method described in Tetrahedron Letters, 36, 8859(1995) in which mercury (II) acetate is used; a method in which dimethyldioxirane is used as a reagent; or other method.

The isolation method for the diaminodicarboxylic acid of the present invention is not particularly limited. When the product is precipitated from the reaction solvent, isolation may be performed by filtration or centrifugal separation. When the product is dissolved in the reaction solvent, removal of the solvent by distillation under reduced pressure or precipitation of the product by addition of a proper solvent followed by filtration or centrifugal separation may be employed as the isolation method. Alternatively, by treatment with a proper acid or base in accordance with the conventional method, a dipolar ionic (zwitterionic) form in which the carboxyl group releases a proton to form -COO- and the amino group combines with a proton to form -NH₃⁺ or a salt with the acid or base may be formed before carrying out the operation described above. The isolation may be carried out by the combination of these methods.

When purification is required, any well-known conventional method may be employed, for example, distillation, recrystallization, column chromatography, sludge treatment, and activated carbon treatment.

The solvent used for recrystallization, column chromatography, sludge treatment, and activated carbon treatment is not particularly limited. Examples of solvents include water; saturated hydrocarbons, such as pentane, hexane, heptane, and cyclohexane; aromatic hydrocarbons, such as benzene, toluene, xylene, and ethyl benzene; halogenated hydrocarbons, such as dichloromethane, chloroform, carbon tetrachloride, dichloroethane, chlorobenzene, and dichlorobenzene; lower alcohols, such as methanol, ethanol, 1-propanol, isopropyl alcohol, and tert-butyl alcohol; glycols, such as ethylene glycol, diethylene glycol, and propylene glycol; ethers, such as ethylene glycol dimethyl ether, 1,3-dioxane, 1,4-dioxane, tetrahydrofuran, dimethyl ether, diethyl ether, diisopropyl ether, and dibutyl ether; amides, such as formamide and N,N-dimethylformamide; nitriles, such as acetonitrile; ketones, such as acetone and methyl ethyl ketone; carboxylic acids, such as formic acid and acetic acid; esters, such as methyl acetate and ethyl acetate; sulfur-containing solvents, such as dimethyl sulfoxide; and 1,3-dimethyl-2-imidazolidinone.

Additionally, aqueous ammonia, hydrochloric acid, an aqueous solution of acetic acid, or the like may be used as water.

These solvents may be used alone or in combination of two or more. When used as a mixture, any mixing ratio is acceptable.

In recrystallization, sludge treatment, or activated carbon treatment, the amount of the solvent used is 1 to 1,000 g and preferably 1 to 300 g relative to 1 g of the diaminodicarboxylic acid.

A method may also be employed in which the diaminodicarboxylic acid of the present invention is transformed into a derivative with a protected carboxyl group or a protected amino group, and the purification process is then performed, followed by deprotection. Examples of the derivative with the protected carboxyl group include ester forms, such as a dimethyl ester form, a diethyl ester form, and a dibenzyl ester form. Examples of the derivative with the protected amino group include a bis(benzyloxycarbonylamino) form and a bis(tert-butoxycarbonylamino) form.

It is also possible to produce the diaminodicarboxylic acid of the present invention by transforming the acid dianhydride represented by general formula (5) into a hydroxamic acid and then by carrying out Lossen rearrangement. Herein, Lossen rearrangement is a reaction in which, by heating a hydroxamic acid, an alkali salt thereof, or an acyl compound thereof, an isocyanic ester and an amine are derived. For example, in a process described in Synthetic Communication, 22, 3067(1992), hydroxylamine and p-toluenesulfonyl chloride are used as reagents, and in a process described in Synthesis, 1143(1990), hydroxylamine-O-sulfonic acid is used as a reagent.

### [Starting material for resin and additive for resin prepared using diaminodicarboxylic acid (salt)]

Starting materials for resins and additives for resins prepared by using diaminodicarboxylic acids and their salts of the present invention will now be described below.

Diaminodicarboxylic acids and their salts of the present invention are mainly used as starting materials for resins, such as polyimides, polyisoimides, and polyamides, and as additives for resins.

Polyimides and polyisoimides are produced by reacting the diaminodicarboxylic acids of the present invention with aromatic acid anhydrides or aliphatic acid anhydrides.

As a starting monomer for a thermosetting resin composition, the diaminodicarboxylic acid of the present invention may be used together with a bismaleimide represented by general formula (12): 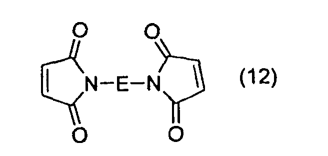 wherein E is a divalent group.

Examples of the divalent group represented by E include, but are not limited to, a p-phenylene group, a m-phenylene group, an o-phenylene group, and groups represented by the following formulae: 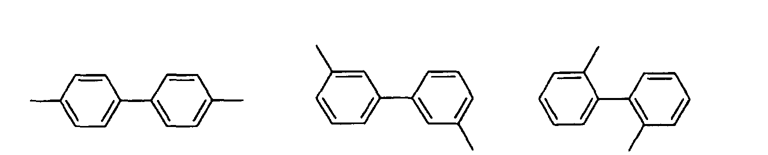 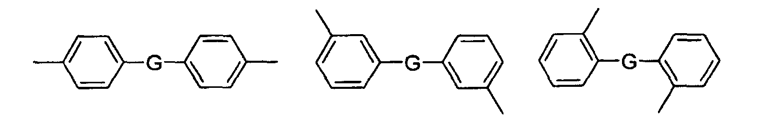 wherein G is an oxygen atom, a sulfur atom, -CH₂-, -C(CH₃)₂-, -SO₂-, -CO-, -NHCO-, or -C(CF₃)₂-. An organic or inorganic filler may be incorporated in the thermosetting resin composition to increase the strength. A prepreg resin may be prepared by impregnating an organic or inorganic textile fabric with the thermosetting resin composition.

As described above, the diaminodicarboxylic acid of the present invention can be used alone in various applications and can also be combined with various other organic compounds, inorganic compounds, or resins to form resins.

When the diaminodicarboxylic acid of the present invention is used as a starting material for resins or an additive for resins, it is possible to give resins having superior physical properties, such as heat resistance, optical characteristics, mechanical characteristics, electrical characteristics, chemical stability, low hygroscopicity, and high formability, compared to conventional amines.

The present invention will be described more in detail based on the examples below. However, it is to be understood that the present invention is not limited thereto.

### (Example 1) Production of diaminobicyclo[2.2.2]oct-7-enedicarboxylic acid dihydrochloride (dihydrochloride of compound represented by general formula (8), wherein one of R¹ and R² is a carboxyl group, and the other is an amino group; one of R³ and R⁴ is a carboxyl group, and the other is an amino group; and each of R⁵ and R⁶ is a hydrogen atom.)

### (1) Synthesis of bicyclo[2.2.2]oct-7-ene-2,3,5,6-tetracarboxydiimide

Ammonia gas was introduced into a suspension of 124.10 g of bicyclo[2.2.2]oct-7-ene-2,3,5,6-tetracarboxylic dianhydride in N,N-dimethylformamide (500 g) at 90°C for 45 minutes. Stirring was performed for 30 minutes at the same temperature, followed by cooling. Ethyl acetate (300 g) was added thereto, and the precipitate was obtained by filtration. To the precipitate was added 300 g of 1 mol/L hydrochloric acid, and stirring was performed for 30 minutes. The substance obtained by filtration was washed with methanol, followed by drying under reduced pressure. The intended substance (98.53 g) was thereby obtained as white crystals.
¹H NMR(OMSO-d₆)δ2.96-3.08(4H,m), 3.19-3.32(2H,m), 6.05-6.15(2H,m).
Melting point: 300°C or more

### (2) Synthesis of diaminobicyclo[2.2.2]oct-7-enedicarboxylic acid dihydrochloride

To a mixture of 12.31 g of bicyclo[2.2.2]oct-7-ene-2,3,5,6-tetracarboxydiimide and 30 g of 30% by weight sodium hydroxide aqueous solution, 150 g of sodium hypochlorite (effective chlorine concentration 5% by weight) was added dropwise for 1 hour while cooling with ice. After stirring for 3 hours at the same temperature, 3.95 g of sodium thiosulfate was added thereto, and water was then removed by distillation. 1 mol/L Hydrochloric acid (100 g) was added thereto, followed by stirring. After water was removed by distillation, hot isopropyl alcohol was added thereto. After insolubles were filtered off, the filtrate was evaporated to dryness. The intended substance (8.33 g) was thereby obtained as light yellow solid.
¹H NMR(D₂O)δ3.03-4.10(6H,m), 6.20-6.75(2H,m)
Melting point: 120°C (decomposition)

### (Example 2) Production of dicarbamoylbicyclohexyldicarboxylic acid (compound represented by general formula (9), wherein one of R¹ and R² is a carboxyl group, and the other is a carbamoyl group; one of R³ and R⁴ is a carboxyl group, and the other is a carbamoyl group; and B is a single bond.)

Ammonia gas was introduced into a suspension of 3.1 g of bicyclohexyl-3,3',4,4'-tetracarboxylic dianhydride in N,N-dimethylformamide (20 g) for 30 minutes while cooling with ice. Stirring was performed for 30 minutes at the same temperature and for another 2 hours at room temperature. After cooling with ice, the precipitate was removed by filtration, and 12 g of 1 mol/L hydrochloric acid was added thereto, followed by stirring for 30 minutes. The solid obtained by filtration was washed with water and methanol, followed by drying under reduced pressure. The intended substance (2.03 g) was thereby obtained as white crystals.
¹H NMR(DMSO-d₆)δ0.88-1.22(4H,m), 1.35-1.65(6H,m), 1.70-1.93(2H,m), 1.95-2.12(2H,m), 2.12-2.26(2H,m), 2.96(2H,s), 6.70-6.94(2H,m), 7.09(2H,s), 12.07(2H,s).
Melting point: 225°C

### (Example 3) Production of diaminobicyclohexyldicarboxylic acid (compound represented by general formula (9), wherein one of R¹ and R² is a carboxyl group, and the other is an amino group; one of R³ and R⁴ is a carboxyl group, and the other is an amino group; and B is a single bond.)

Dicarbamoylbicyclohexyldicarboxylic acid (16.90 g) was dissolved in 7% by weight sodium hydroxide aqueous solution (184.42 g). And to this solution, 105.60 g of sodium hypochlorite aqueous solution (effective chlorine concentration 5% by weight) was added dropwise at 10°C or less for 30 minutes. Stirring was then performed at 35°C for 6 hours and at room temperature for 17 hours. The solution was cooled to 15°C, and its pH was adjusted to 6.8 by adding 23.02 g of concentrated hydrochloric acid. The precipitated solid was obtained by filtration and washed with water, followed by drying under reduced pressure. The intended substance (6.78 g) was thereby obtained as white solid.
¹H NMR(D₂O, containing DCl)δ0.80-1.00(2H,m), 1.20-1.65(8H,m), 1.66-1.89(2H,m), 2.00-2.26(2H,m), 2.90-3.10(2H,m), 3.20-3.45(2H,m).
MS (ESI) (positive): 285 [M+H]⁺
(negative): 283 [M-H]⁻
Melting point: 275°C or more

### (Example 4) Production of diaminobicyclohexyldicarboxylic acid

A mixture of 1.70 g of dicarbamoylbicyclohexyldicarboxylic acid, 4.3 g of I,I-bis(trifluoroacetoxy)iodobenzene, 5 g of acetonitrile, and 5 g of water was stirred at 60°C for 1 hour. After the mixture was cooled to room temperature, 10 ml of concentrated hydrochloric acid was added thereto, and the precipitate was filtered off. Water (5g) was added to the concentrated residue of the filtrate, and the pH was adjusted to 5.7 by adding 0.1 mol/L sodium hydroxide aqueous solution. After water was removed by distillation, ethanol was added thereto, and the precipitated solid was filtered off. The resultant filtrate was dried under reduced pressure. The intended substance (0.10 g) was thereby obtained as white solid.
¹H NMR(DMSO-d₆)δ0.80-1.50(10H,m), 1.60-2.38(4H,m), 2.77(2H,m), 3.07(2H, m).

### (Example 5) Production of diaminobicyclohexyldicarboxylic acid dihydrochloride

Dicarbamoylbicyclohexyldicarboxylic acid (20.42 g) was dissolved in 5.7% by weight sodium hydroxide aqueous solution (273.68 g). And to this solution, 127.61 g of sodium hypochlorite aqueous solution (effective chlorine concentration 5% by weight) was added dropwise at 10°C or less for 1 hour. Stirring was then performed at 35°C for 1 hour. After the solution was cooled to 10°C, 60.77 g of concentrated hydrochloric acid was added thereto, and the precipitate was filtered off. Ethanol was added to the concentrated residue of the filtrate, and the precipitated solid was removed by filtration. The solid was sludged using ethanol, and a filtrate was obtained by filtration. The solid obtained by filtration was further subjected to the similar treatment five times. The individual filtrates were mixed, and the mixture was dried under reduced pressure. The intended substance (4.52 g) was thereby obtained as white solid.
¹H NMR(DMSO-d₆)δ0.80-1.00(2H,m), 1.20-1.65(8H,m), 1.66-1.89(2H,m), 2.00-2.22(2H,m), 3.00-3.25(4H,m), 8.25(6H,s), 12.9(2H,s).
Melting point: 330°C
MS (ESI) (positive): 285 [M+H]⁺
(negative): 283 [M-H]⁻

### Industrial Applicability

According to the present invention, it is possible to provide a novel diaminodicarboxylic acid which is useful as a starting material for resins or an additive for resins, a synthetic intermediate for the diaminodicarboxylic acid, and a process for producing the diaminodicarboxylic acid.

## Claims

1. A compound represented by general formula (1) or a salt thereof: 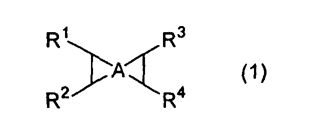 wherein A is a tetravalent aliphatic group having 2 to 20 carbon atoms which may contain at least one of a halogen atom, oxygen atom, and sulfur atom; one of R¹ and R² is a carboxyl group, and the other is an amino group or a carbamoyl group; and one of R³ and R⁴ is a carboxyl group, and the other is an amino group or a carbamoyl group.

2. A compound or a salt thereof according to Claim 1, wherein, in general formula (1), one of R¹ and R² is a carboxyl group, and the other is an amino group; and one of R³ and R⁴ is a carboxyl group, and the other is an amino group.

3. A compound or a salt thereof according to Claim 1, wherein, in general formula (1), one of R¹ and R² is a carboxyl group, and the other is a carbamoyl group; and one of R³ and R⁴ is a carboxyl group, and the other is a carbamoyl group.

4. A compound or a salt thereof according to any one of Claims 1 to 3, wherein, in general formula (1), the group represented by: 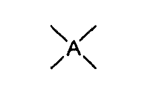 is any one of a group represented by general formula (2): 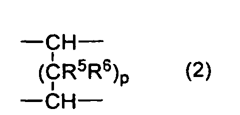 wherein each of R⁵ and R⁶ is independently a hydrogen atom, a halogen atom, or an alkyl group having 1 to 6 carbon atoms; R⁵ and R⁶ may be linked together to form a divalent hydrocarbon group; each of the alkyl group and the divalent hydrocarbon group may be substituted with a halogen atom; and p is an integer of 1 to 3,
a group represented by general formula (3): 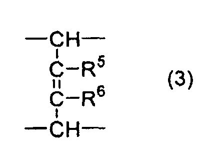 wherein R⁵ and R⁶ are as defined in general formula (2), and a group represented by general formula (4): 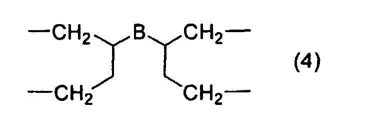 wherein B is a single bond, a divalent hydrocarbon group having 1 to 12 carbon atoms, an oxygen atom, a sulfur atom, -CO-, or -SO₂-; and the divalent hydrocarbon group may be substituted with a halogen atom.

5. A process for producing a diaminodicarboxylic acid represented by general formula (6) or a salt thereof: 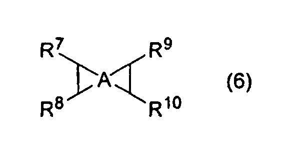 wherein A is a tetravalent aliphatic group having 2 to 20 carbon atoms which may contain at least one of a halogen atom, oxygen atom, and sulfur atom; one of R⁷ and R⁸ is a carboxyl group, and the other is an amino group; and one of R⁹ and R¹⁰ is a carboxyl group, and the other is an amino group, the process comprising the steps of:
reacting an acid dianhydride represented by general formula (5): 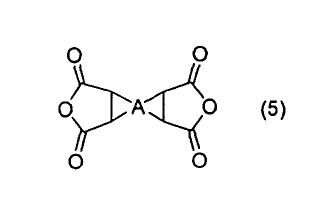
wherein A is as defined in general formula (6), with ammonia; and
subjecting the reaction product to Hofmann rearrangement.
